Europäisches Patentamt

European Patent Office

Office européen des brevets

(11) Publication number: **0 085 083**
**B1**

(12)                    EUROPEAN PATENT SPECIFICATION

(45) Date of publication of patent specification: **23.01.85**    (51) Int. Cl.⁴: **C 12 P 21/02**

(21) Application number: **82902439.7**

(22) Date of filing: **10.08.82**

(86) International application number:
**PCT/DK82/00074**

(87) International publication number:
**WO 83/00504 17.02.83 Gazette 83/05**

(54) **Process for the enzymatic preparation of human insulin.**

(30) Priority: **10.08.81 DK 3545/81**

(43) Date of publication of application:
**10.08.83 Bulletin 83/32**

(45) Publication of the grant of the patent:
**23.01.85 Bulletin 85/04**

(84) Designated Contracting States:
**AT BE CH DE FR GB LI LU NL SE**

(56) References cited:
**EP-A-0 017 938**
**SE-A-81 009 284**

**Peptides 1980, Proceedings of the Sixteenth European Peptide Symposium Helsingoer, DK, Aug 31 - Sep 6, 1980, Copenhagen 1981, Ed. K.BRUNFELDT, Trypsin catalized peptide synthesis: Modification of the B-chain C-terminal region of insulin, GATTNER H-G et al., p. 372-7**

(73) Proprietor: **NORDISK INSULINLABORATORIUM**
**Niels Steensensvej 1**
**DK-2820 Gentofte (DK)**

(72) Inventor: **ANDRESEN, Finn Hede**
**Sommersvej 21**
**DK-3400 Hilleroed (DK)**
Inventor: **BALSCHMIDT, Per**
**Tibberup Allé 20**
**DK-3060 Espergaerde (DK)**
Inventor: **HEJNAES, Kim Ry**
**Bredebovej 29**
**DK-2800 Lyngby (DK)**

(74) Representative: **Patentanwälte Beetz sen. -**
**Beetz jun. Timpe - Siegfried - Schmitt-Fumian**
**Steinsdorfstrasse 10**
**D-8000 München 22 (DE)**

Courier Press, Leamington Spa, England.

# Description

The present invention relates to a novel process for the preparation of a human insulin product being suitable for pharmaceutical insulin compositions wherein a porcine insulin product is treated with carboxypeptidase A or a corresponding enzyme to form a desalanine-B30 insulin product which is subsequently condensed with a L-threonine alkyl ester in the presence of trypsin or an enzyme related thereto and an organic solvent, whereafter the insulin ester formed is hydrolyzed to human insulin.

Insulin is an indispensable medicine for the treatment of diabetes. It would be natural to treat human beings with pharmaceutical compositions prepared from insulin of human origin. However, the number of diabetics and the individual need for insulin are disproportionate to the available amount of raw material (human pancreas).

Therefore, for practical reasons therapeutical preparations prepared from bovine and porcine insulin are used. However, to a larger or smaller extent, these insulins give rise to the formation of antibodies in the human body, which involves a reduced effect of the further insulin treatment.

This disadvantage is supposed to be caused partly by impurities in the bovine and porcine insulin, partly by the alien nature, since the amino acid sequence of bovine insulin differs from that of the human insulin in the positions A8, A10, and B30, whereas porcine insulin only differs from human insulin in the B30-position.

The problem of the formation of antibodies has been substantially reduced since the introduction of therapeutical preparations prepared from chromatographically purified bovine and porcine insulin. However, the formation of antibodies may still occur. It is believed that this can be remedied by using pharmaceutical compositions prepared from insulin of the same amino acid sequence as human insulin.

It is known to prepare insulin synthetically, vide US patent 3 903 068 and Hoppe-Seyler's Z. Physiol. Chem., *357*, 759—767 (1976).

These processes comprise condensing a desoctapeptide-(B23-30) porcine insulin with a synthetic octapeptide corresponding to the positions B-23-30 in human insulin. However, in one of these processes an alkaline hydrolysis is carried out, which is accompanied by unfavourable side reactions. In another process a non-specific reaction is comprised giving rise to many side reactions and demanding complicated purification procedures. Consequently, these processes are not suitable for use on an industrial scale.

Moreover, US patent 3 276 961 discloses a process for the preparation of human insulin from other animal insulins by the action of an enzyme, e.g. carboxypeptidase A or trypsin, in the presence of threonine.

However, by this known process it is not possible to prepare human insulin to any appreciable extent. This is probably due to the fact that trypsin and carboxypeptidase A hydrolyze not only the lysyl-alanine peptide bond (B29—B30), but also other positions in insulin under the working conditions. Trypsin preferably hydrolyzes the arginyl-glycine peptide bond (B22—B23) rather than the lysyl-alanine bond (B29—B30). However, carboxypeptidase A cannot exclusively split off the alanine at the C-terminal of the B-chain without also splitting off asparagine at the C-terminal of the A-chain. It has later been shown that a specific condition, i.e. reaction in an ammonium bicarbonate buffer solution, is necessary in order to hinder the asparagine release, cf. Hoppe-Seyler's Z. Physiol. Chem., *359*, 799—802 (1978). Moreover, a considerable peptide formation scarcely occurs, since the rate of the hydrolysis reaction is higher than that of the peptide synthesis at the working conditions.

Finally, Nature Vol. 280, 2nd August, 1979, 412—413, Biochem. & Biophys. Res. Commun., 29th January, 1980, 92(2), 396—402, and Danish published Patent Specification 146 482 (European published Patent Application 0 017 938) disclose a method of exchanging the B-30 amino acid, in which treatment of porcine insulin with carboxypeptidase A, to which an inhibitor (diisopropyl fluorophosphate) has been added, at 25°C for 8 h in the presence of ammonium bicarbonate buffer results in a desalanine insulin (DAI), which is isolated. Thereafter the coupling reaction is carried out by adding a solution of trypsin in 0.5 M borate buffer solution and tosyl-L-phenylalanine chloromethyl ketone (TPCK) to a solution of DAI and threonine butyl ester (Thr-OBu$^t$) in a solvent containing an organic solvent mixture of a high concentration (about 60%) consisting of a mixture of DMF and ethanol, whereafter the reaction proceeds at 37°C for 20—48 h to form (B30-Thr-OBu$^t$) porcine insulin, which is isolated. Finally, the butyl ester protecting group is split off with trifluoroacetic acid in the presence of anisole.

Investigations have however shown that the latter known method results in a human insulin product being unsuitable for the preparation of pharmaceutical compositions since it contains enzyme impurities and accordingly is unsuitable for use in the preparation of the necessary pharmaceutical preparations of protracted activity. Furthermore, the product is mutagenic in the "Ames Test", in contradistinction to human insulin and porcine insulin prepared from pancreas of human and porcine origin, respectively. Finally, it contains impurities, which are difficult to remove by the methods generally used.

It has now been found that it is possible to prepare a highly pure human insulin product free of the above-mentioned disadvantages of the human insulin products prepared by the known processes.

According to the invention, the coupling of desalanine-B30 insulin (DAI) with L-threonine alkyl ester is carried out using a specific sequence of mixing the two reactants, enzyme and reaction medium without using a buffer. Thereby the coupling reaction is completed in a surprisingly short time. A mixture of a lower alkanol and water of a pH value in the range of from about 5.5 to about 7.5 is used as reaction medium. The insulin ester thus prepared is then converted into human insulin in a lenient manner, partly by an appropriate choice of the alkyl moiety of the ester, partly by carrying out the hydrolysis in an aqueous medium of a pH value in the range of from 8.5 to 10.5. Thus, the addition of enzyme, trifluoroacetic acid and anisole is avoided.

Accordingly, the process of the invention is characterized by suspending the desalanine-B30 insulin product in a lower alkanol, mixing a solution of the L-threonine alkyl ester and trypsin or an enzyme related thereto in water adjusted to a pH value of from about 5.5 to about 7.5 without addition of a buffer with the suspension and leaving the resulting mixture for up to 30 min, and by carrying out the hydrolysis of the insulin ester in an aqueous medium of a pH value in the range of from about 8.5 to about 10.5.

When the process of the invention is carried out on a large scale such as industrial scale using e.g. 50 g or more of the starting insulin product a preferred embodiment of the invention comprises dissolving separately trypsin or an enzyme related thereto in part of the solution of the L-threonine alkyl ester in water, adding the suspension of the desalanine-B30 insulin product to the remaining part of the solution of the L-threonine alkyl ester in water, adjusting the temperature and pH value of the resulting mixture and then adding the separately prepared solution of trypsin or an enzyme related thereto in part of the solution of the L-threonine alkyl ester in water. In this case it is preferred that the temperature and the pH value of the resulting mixture are adjusted to 10—15°C. and 5.8—6.2, respectively. Moreover, it is preferred to dissolve separately trypsin or an enzyme related thereto in 10—20% by weight of the solution of the L-threonine alkyl ester in water.

It is pointed out that the condensation reaction is carried out without the addition of a buffer, the buffering capacity of the reactants being sufficient under the conditions given according to the invention to maintain a pH in the range of 5.5 to 7.5.

The above features are essential features of the process, and they are decisive of the possibility of carrying out the reaction in a very short time (reduced by a factor 100) on an industrial scale.

In the condensation reaction the reaction time is dependent on the reaction conditions. Reaction times from about 5 min to about 30 min, preferably from about 5 to about 15 min, are used.

As a consequence of the short reaction times in the condensation reaction no appreciable side reactions occur, and moreover, it is not necessary to use enzymes treated with tosyl-L-phenylalanine chloromethyl ketone (TPCK).

In a particularly advantageous embodiment of the process of the invention raw porcine insulin, e.g. insulin salt cake, is used as starting material. It is worth noting that when using raw porcine insulin in the process of the invention it is possible to obtain a high yield of human insulin corresponding to the yield which might be obtained when preparing chromatographically purified porcine insulin, when, in both cases, the total yields are calculated on the amount of pancreas used.

It is surprising that the use of raw porcine insulin as starting material does not give rise to difficulties in the chromatographical separation of the obtained human insulin ester from the enzyme used and unreacted desalanine-B30 insulin.

The enzyme used in the condensation of the process of the invention must be capable of splitting lysine carbonyl peptide bonds, and thus use can be made of trypsin or enzymes related thereto, e.g. a chromobacter protease I, the preparation and properties of which are described by Masaki et al., Agric. Biol. Chem., *42*, 1443—1445 (1978). It is unnecessary to use enzymes treated with tosyl-L-phenylalanine chloromethyl ketone (TPCK) to eliminate a possible contamination with chymotrypsin-like enzymes due to the short reaction times mentioned above and the poor reactivity of such enzymes in the reaction mixture used.

The enzyme can be used in dissolved form, but can also be bound to an insoluble matrix, e.g. agarose or polyacrylamide or similar polymeric substances.

The condensation reaction is carried out under conditions where the enzymatically catalyzed hydrolysis is sufficiently suppressed for the peptide forming reaction to proceed. As mentioned above, the pH value must be between 5.5 and 7.5. The temperature is usually in the range of 0 to 50°C, preferably from 10 to 37°C.

In the condensation reaction the concentration of the reactants, i.e. des-B30 insulin and L-threonine alkyl ester, should be high, and moreover, the L-threonine alkyl ester used should be employed in a large excess up to a molar ratio of 200:1, preferably in the range of 20:1 to 100:1.

The condensation reaction is carried out in the presence of water-miscible lower alkanols or mixtures thereof, whereby the hydrolysis reaction is hindered, and the solubility of the reactants is improved. The concentration of lower alkanols should be selected in the range of 20 to 90%, preferably 30 to 80%, calculated on the total volume of the reaction mixture.

When the condensation reaction is complete, the insulin-like proteins are separated from the remaining components by gel chromatography, whereupon human insulin ester is separated from unreacted starting material by anion exchange chromatography. The unreacted starting material may possibly be reused in the process.

The collected fractions from the anion exchange containing human insulin ester are desalted, whereafter the pH value of the collected eluate is adjusted to about 9.5 by means of NaOH. After 24—48 h at ambient temperature pure human insulin is isolated by crystallization or other usual methods.

The hydrolysis of the insulin alkyl ester proceeds smoothly at a pH value of from about 8.5 to about 10.5 in an aqueous solution. This has the effect that, after the hydrolysis, the hydrolysis mixture can easily be worked up in a conventional manner and that the isolated human insulin is obtained in a high purity.

Thus, the human insulin prepared by the process of the invention is well-suited for the preparation of pharmaceutical insulin compositions since it contains no proteolytic impurities, for which reason it can be used for preparations having protracted activity, since it behaves in the same way as human insulin and porcine insulin prepared from Pancreas of human and porcine origin in the "Ames Test" mentioned above, and since it contains no impurities being difficult to remove by means of chromatographical methods generally used. .

Moreover, in a preferred embodiment, where raw insulin is used as starting material, a yield of human insulin is obtained which in all essentials is equal to the yield of highly purified porcine insulin which can be obtained from the same amount of raw insulin. Thus, there is question of a method which is industrially as well as clinically satisfactory to a hitherto unknown degree.

The process of the invention is further illustrated by means of the following Examples.

Example 1

Porcine insulin in the form of raw insulin (salt cake) corresponding to 2000 mg of porcine insulin was dissolved in 200 ml of 0.2 M aqueous $NH_4HCO_3$ solution (pH 8.4). To the solution 20 mg of carboxypeptidase A in the form of an aqueous solution of a concentration of about 5 mg/ml were added. The mixture was 5 min carefully stirred and then left for 2.5 h at 20°C. Subsequently, the reaction mixture was freeze-dried.

Determination of released alanine by means of amino acid analysis showed a cleavage yield of 92%.

The freeze-dried powder was suspended in 23.4 ml of 96% ethanol followed by the addition of a solution of 4.0 g of L-threonine methyl ester and 200 mg of trypsin in 14.0 ml of distilled water adjusted to a pH value of 6.50 with 5 N hydrochloric acid. After careful mixing the mixture was left for 15 min at 35°C. Immediately thereafter the reaction was stopped by adjusting first the pH value to 2.5 with 0.1 N hydrochloric acid and the volume to 100 ml by means of distilled water.

High pressure liquid chromatographical analysis of the reaction mixture showed a yield of human insulin ester of 80%.

The reaction mixture was purified by gel chromatography on a column of Sephadex® G-50 Superfine (8×80 cm) in 1 M acetic acid. The fraction containing human insulin ester and unreacted desalanine-B30 insulin was freeze-dried.

Yield: 1612 mg of product mixture.

Thereafter the product mixture was ion-exchanged at 4°C on a column of DEAE cellulose (Whatmann® DE-52) (9×23 cm), equilibrated with 140 ml/h of a buffer consisting of 0.02 M tris and 7 M urea, adjusted to a pH value of 8.1 with hydrochloric acid. When the charging of the product was complete, the column was eluted for 2.5 h using the above-mentioned buffer solution, then for 2 h using the above-mentioned buffer in admixture with 0.0045 moles of sodium chloride per liter and finally for 12 h using the former buffer in admixture with 0.011 moles of sodium chloride per liter.

The eluate contained two proteinaceous main fractions. The fraction eluted at first was identified by high pressure liquid chromatography as being human insulin ester and the fraction eluted thereafter as being desalanyl insulin.

The collected human insulin ester fraction was desalted on a column of Sephadex® G-25 in 0.1 M sodium acetate (pH 8.0) at 4°C, whereafter the pH value was adjusted to 9.5 with 1 N NaOH solution. The fraction was left at 25°C for 24 h.

950 mg of pure human insulin were obtained, identified by amino acid analysis and high pressure chromatography.

Example 2

Porcine insulin in the form of raw insulin (salt cake) corresponding to 66.3 g of porcine insulin was dissolved in 6000 ml of 0.2 M ammonium acetate, pH 8.4. To the solution 600 mg of carboxypeptidase A in the form of an aqueous solution of a concentration of about 5 mg/ml were added. The mixture was carefully stirred for 3 h at 20°C.

The desalanine insulin was precipitated by addition of 15% (w/v) NaCl solution.

The isolated air-dried precipitate was carefully poured into 770 ml of 96% ethanol thus forming a suspension of desalanine insulin in ethanol (solution 1).

120 g of Thr-O-Me · HCl was dissolved in 275 ml of distilled water and the pH value adjusted to 5.8 with 5 M NaOH (solution 2).

To 60 ml of solution 2 there were added 600 mg of trypsin (porcine), brought into solution by careful stirring (solution 3).

Solution 1 and 2 were mixed by slowly pouring solution 1 into solution 2 while stirring. The pH value was adjusted to 5.8 with 5 M NaOH. After mixing, the mixture was cooled to 10°C in a thermostatically controlled jar.

The reaction took place after addition of solution 3 to the above mentioned mixture of desalanine insulin and Thr-O-Me for 10 min.

Immediately thereafter the reaction was stopped by adjusting the pH value to 2.5 with 0.1 N HCl and adjusting the volume to 2400 ml by means of distilled water.

High pressure liquid chromatographical analysis of the reaction mixture showed a yield of human insulin ester of 87%.

The reaction mixture was worked up as described in Example 1 resulting in a highly purified product (25.4 g) of semisynthetic human insulin suitable for the preparation of pharmaceutical insulin compositions.

## Claims

1. A process for the preparation of human insulin by

— treating a porcine insulin product with carboxypeptidase A or a corresponding enzyme to form a desalanine-B30 insulin product,
— subsequent condensation with an L-threonine alkyl ester in the presence of trypsin or an enzyme related thereto and an organic cosolvent, and
— final hydrolysis of the insulin ester obtained to form human insulin, characterized by
— suspending the desalanine-B30 insulin product in a lower alkanol,
— mixing a solution of the L-threonine alkyl ester and trypsin or an enzyme related thereto in water adjusted to a pH value of from about 5.5 to about 7.5 without addition of a buffer with the suspension and leaving the resulting mixture for up to 30 min and
— hydrolysing the insulin ester in an aqueous medium of a pH value in the range of from about 8.5 to about 10.5.

2. A process according to claim 1, characterized by separately dissolving trypsin or an enzyme related thereto in part of the solution of the L-threonine alkyl ester in water, adding the suspension of the desalanine-B30 insulin product to the remaining part of the solution of the L-threonine alkyl ester in water, adjusting the temperature and pH value of the resulting mixture and then adding the separately prepared solution of trypsin or an enzyme related thereto in part of the solution of the L-threonine alkyl ester in water.

3. A process according to claim 2, characterized by separately dissolving trypsin or an enzyme related thereto in 10—20% by weight of the solution of the L-threonine alkyl ester in water.

4. A process according to one of claims 1—3, characterized in that raw porcine insulin is used as said porcine insulin product.

5. A process according to one of claims 2—4, characterized in that the temperature and the pH value of the resulting mixture are adjusted to 10—15°C and 5.8—6.2, respectively.

6. A process according to one of claims 1—5, characterized in that the condensation reaction is carried out about 5 to about 15 min.

7. A process according to one of claims 1—6, characterized in that the desalanine-B30 insulin product is isolated without separation of the employed carboxypeptidase A or corresponding enzyme.

8. A process according to one of claims 1—7, characterized in that L-threonine methyl ester is used as said L-threonine alkyl ester.

9. A process according to one of claims 1—8, characterized in that ethanol is used as said lower alkanol.

## Patentansprüche

1. Verfahren zur Herstellung eines Human- insulins durch

— Behandlung eines Schweineinsulinproduktes mit Carboxypeptidase A oder einem entsprechenden Enzym zu einem Desanilin-B30-Insulinprodukt,
— anschliessender Kondensation mit einem L-Threonin-alkylester in Gegenwart von Trypsin oder einem damit verwandten Enzym und einem organischen Colösungsmittel, und
— Endhydrolyse des erhaltenen Insulinesters zu Humaninsulin, dadurch gekennzeichnet, daß
— das Desanilin-B30-Insulinprodukt in einem niederen Alkanol suspendiert wird,
— die Suspension mit einer Lösung des L-Threonin-alkylesters und des Trypsins oder eines damit verwandten Enzyms in Wasser, das auf einen pH-Wert von etwa 5,5 bis etwa 7,5 ohne Zugabe eines Puffers eingestellt worden ist, vermischt wird und das entstandene Gemisch bis zu 30 min stehen gelassen wird, und
— der Insulinester in einem wäßrigen Medium mit einem pH-Wert von etwa 8,5 bis etwa 10,5 hydrolysiert wird.

2. Verfahren nach Anspruch 1, dadurch gekennzeichnet, daß das Trypsin oder ein damit verwandtes Enzym in einem Teil der Lösung des L-Threonin-alkylesters in Wasser getrennt gelöst wird, die Suspension des Desanilin-B30-Insulinproduktes zu dem Rest der Lösung des L-Threonin- alkylesters in Wasser zugegeben wird, die Temperatur und der pH-Wert des entstandenen Gemisches eingestellt werden und dann die getrennt hergestellte Lösung des Trypsins oder eines damit verwandten Enzyms in einem Teil der Lösung des L-Threonin-alkylesters in Wasser zugegeben wird.

3. Verfahren nach Anspruch 2, dadurch gekennzeichnet, daß das Trypsin oder ein damit verwandtes Enzym getrennt in 10 bis 20 Gew.-% der Lösung des L-Threonin-alkylesters in Wasser gelöst wird.

4. Verfahren nach einem der Ansprüche 1 bis 3, dadurch gekennzeichnet, daß als Schweineinsulinprodukt Schweine-Rohinsulin verwendet wird.

5. Verfahren nach einem der Ansprüche 2 bis 4, dadurch gekennzeichnet, daß die Temperatur und der pH-Wert des entstandenen Gemisches auf 10 bis 15°C bzw. auf 5,8 bis 6,2 eingestellt werden.

6. Verfahren nach einem der Ansprüche 1 bis 5, dadurch gekennzeichnet, daß die Kondensationsreaktion etwa 5 bis 15 min lang durchgeführt wird.

7. Verfahren nach einem der Ansprüche 1 bis 6, dadurch gekennzeichnet, daß das Desanilin-B30-Insulinprodukt ohne Abtrennung der verwendeten Carboxypeptidase A oder des entsprechenden Enzyms isoliert wird.

8. Verfahren nach einem der Ansprüche 1 bis 7, dadurch gekennzeichnet, daß als L-Threonin-alkylester L-Threonin-methylester verwendet wird.

9. Verfahren nach einem der Ansprüche 1 bis 8, dadurch gekennzeichnet, daß als niederes Alkanol Ethanol verwendet wird.

**Revendications**

1. Procédé de préparation d'une insuline humaine, par

— traitement d'un produit de type insuline porcine par de la carboxypeptidase A, ou par une enzyme correspondante, pour former un produit de type désalanine-B30 insuline,
— condensation subséquente avec un ester alkylique de L-thréonine en présence de trypsine, ou d'une enzyme qui lui est apparentée, et d'un cosolvant organique, et
— hydrolyse finale de l'ester d'insuline obtenu pour former de l'insuline humaine, procédé caractérisé en ce que:
— on met le produit de type désalanine-B30 insuline en suspension dans un alcanol inférieur;
— on mélange une solution de l'ester alkylique de L-thréonine et de trypsine, ou d'une enzyme qui lui est apparentée, dans de l'eau dont le pH a été ajusté à une valeur comprise entre environ 5,5 à environ 7,5, sans addition d'un tampon, avec la suspension et on laisse le mélange résultant pendant un temps pouvant aller jusqu'à 30 min, et
— on hydrolyse l'ester d'insuline dans un milieu aqueux dont le pH se situe entre environ 8,5 et environ 10,5.

2. Procédé selon la revendication 1, caractérisé en ce que:

— on dissout séparément la trypsine, ou une enzyme qui lui est apparentée, dans une partie de la solution de l'ester alkylique de L-thréonine dans de l'eau,
— on ajoute la suspension du produit de type désalanine-B30 insuline à la partie restante de la solution de l'ester alkylique de L-thréonine dans de l'eau,
— on ajuste la température et le pH du mélange résultant, puis
— on ajoute la solution séparément préparée de trypsine, ou d'une enzyme qui lui est apparentée, dans une partie de la solution aqueuse de l'ester alkylique de L-thréonine.

3. Procédé selon la revendication 2, caractérisé en ce qu'on dissout séparément la trypsine, ou une enzyme qui lui est apparentée, dans 10 à 20% en poids de la solution aqueuse de l'ester alkylique de L-thréonine.

4. Procédé selon l'une des revendications 1 à 3, caractérisé en ce qu'on utilise de l'insuline porcine brute comme produit de type insuline porcine.

5. Procédé selon l'une des revendications 2 à 4, caractérisé en ce qu'on ajuste la température entre 10 et 15°C et le pH du mélange résultant entre 5,8 et 6,2, respectivement.

6. Procédé selon l'une des revendications 1 à 5, caractérisé en ce qu'on effectue la réaction de condensation durant environ 5 à environ 15 min.

7. Procédé selon l'une des revendications 1 à 6, caractérisé en ce qu'on isole le produit de type désalanine-B30 insuline sans séparer la carboxypeptidase A, ou une enzyme correspondante, que l'on a utilisée.

8. Procédé selon l'une des revendications 1 à 7, caractérisé en ce qu'on utilise de l'ester méthylique de L-thréonine à titre de l'ester alkylique de L-thréonine.

9. Procédé selon l'une des revendications 1 à 8, caractérisé en ce qu'on utilise de l'éthanol comme alcanol inférieur.